# FASCICULE DE BREVET EUROPEEN

(11) **EP 0 374 053 B1**
(45) Date de publication et mention de la délivrance du brevet: **16.03.1994**
(21) Numéro de dépôt: 89403495.8
(22) Date de dépôt: 15.12.1989
(51) Int. Cl.: A61K 39/42

(54) **Médicament pour le traitement ou la prévention par immunisation passive de l'infection par le virus HIV et procédés de préparation**
Arzneimittel für die Behandlung oder die Verhütung von Infektionen mit HIV durch Immunisierung, und Verfahren zur Herstellung
Medicine for the treatment or prevention by immunisation of an infection by HIV, and preparation processes

(30) Priorité: 15.12.1988 FR 8816580
(43) Date de publication de la demande: 20.06.1990
(73) Titulaire: PASTEUR MERIEUX SERUMS ET VACCINS, 69007 Lyon (FR)
(72) Inventeur: Grandgeorge, Michel, F-69670 Vaugneray (FR); Pelloquin, François, F-69008 Lyon (FR)
(74) Mandataire: Bernasconi, Jean

(56) Documents cités:
- EP-A- 0 234 405
- EP-A- 0 260 691
- WO-A-89/01339
- NATURE, vol. 316, no. 6023, juillet 1985, Londres (GB); M.ROBERT-GUROFF et al., pp. 72-74#
- NATURE, vol. 316, no. 6023, 04 juillet 1985, Londres (GB); R.A.WEISS et al., pp. 69-72#

## Description

La présente invention a trait à un médicament pour le traitement ou la prévention, par immunisation passive, de l'infection par le virus HIV.

On a déjà envisagé l'utilisation de gammaglobulines spécifiques anti-HIV d'origine humaine obtenues à partir de donneurs séro-positifs pour la séroprophylaxie et le traitement de l'infection par le virus HIV (virus de l'immunodéficience humaine). Voir Immunoglobulin preparation for HIV-infected patients - P.L. YAP et P.E. WILLIAMS - Vox Sanguinis 55 : 65-74 (1988).

Cependant, ces gammaglobulines contiennent un grand nombre d'anticorps anti-HIV différents, parmi lesquels on peut citer des anticorps dirigés contre les protéines de la paroi du virus (dans le cas de HIV1 : glycoprotéines gp120 et gp41 ou leur précurseur gp160), les anticorps dirigés contre les protéines du noyau viral (protéines p24, p18 et p15) ou anticorps dirigés contre les enzymes virales, notamment la transcriptase inverse (RT). De plus, les donneurs séropositifs pourraient également posséder des anticorps dirigés contre la protéine marqueur des lymphocytes T4 (protéine CD4) par suite d'une auto-immunisation déclenchée par l'interaction de la protéine gp120 du virus avec la protéine CD4 qui constitue son récepteur naturel au niveau des lymphocytes T4.

Or, les anticorps dirigés contre les protéines de l'enveloppe du virus sont soupçonnés d'être "facilitants" au lieu de protéger contre l'infection virale, du fait qu'ils formeraient un complexe immun avec le virus et provoqueraient sa phagocytose par les macrophages, conduisant ainsi à une infection des macrophages qui constitueraient un réservoir pour le virus HIV.

Par ailleurs, les anticorps dirigés contre la protéine CD4 seraient également soupçonnés d'avoir un effet néfaste lors de l'infection HIV en provoquant la cytolyse des lymphocytes T4 et en participant ainsi à l'établissement du syndrome d'immunodéficience acquise (SIDA), manifestation clinique de l'infection par le virus HIV.

De plus, il a été observé que l'apparition des signes cliniques du SIDA chez une personne séropositive, auparavant asymptomatique, est accompagnée d'un effondrement des anticorps anti-p24 et anti-RT alors que le titre d'anticorps neutralisants in vitro n'est pas modifié. Voir : Prospects for the control of AIDS by immunizing seropositive individual : J. SALK, Nature 327 : 473-76, 11 juin 1987.

D'autres détails sur les processus de l'infection par le virus HIV apparaissent dans : Replication of the human immunodeficiency virus - Strategies for inhibition. B.M. PETERLIN and P.A. LUCIW, Biotechnology 6 : 794-799, juillet 1988.

La présente invention se propose de fournir un médicament pour le traitement ou la prévention de l'infection par le virus HIV en utilisant des immunoglobulines humaines hyperimmunes anti-HIV.

L'invention a pour objet un médicament constitué par ou comprenant une préparation de gammaglobulines IgG et/ou IgM d'origine humaine hyperimmunes anti-HIV dépourvue d'anticorps dirigés contre les glycoprotéines d'enveloppe du virus HIV et/ou d'anticorps anti-CD4.

Le médicament selon l'invention est avantageusement conditionné pour être administré par voie parentérale, notamment injection intramusculaire ou intraveineuse, de la même façon que les préparations usuelles d'immunoglobulines.

L'invention a également pour objet des procédés de préparation de ce médicament.

Selon un tel procédé, on collecte du plasma, du sang ou des placentas de donneurs ou mères séropositifs pour le virus HIV. A partir de ce matériau, on obtient, par des procédés d'isolation ou de fractionnement connus, la préparation d'immunoglobulines totales IgG et/ou IgM, et on traite cette préparation pour en éliminer les anticorps anti-enveloppe HIV et/ou anti CD4.

Dans un autre mode de mise en oeuvre du procédé selon l'invention, on extrait sélectivement, à partir du plasma, du sang ou des placentas collectés, un ou plusieurs anticorps dirigés contre le virus HIV mais non dirigés contre les glycoprotéines d'enveloppe et/ou contre CD4.

En particulier, on peut extraire un ou plusieurs des anticorps anti-core, anti-p24, 25 anti-p55, anti-p18, anti-transcriptase inverse ou anti nef.

L'extraction peut se faire, par exemple, en fixant le ou les antigènes HIV naturels, ou des antigènes présentant l'épitope pertinent, obtenu par synthèse chimique ou expression dans un système recombinant, sur un support, par exemple sur un gel d'agarose.

Lorsque l'on extrait sélectivement, des immuglobulines collectées, l'anticorps en question, on obtient l'avantage d'une concentration plus élevée et d'une standardisation du titre et de la composition des immunoglobulines extraites à partir des immunoglobulines collectées. On peut ensuite, éventuellement, mélanger les immunoglobulines sélectivement extraites et obtenir des titres standardisés en anticorps.

Lorsque l'on élimine sélectivement, des immunoglobulines collectées, les anticorps anti-enveloppe HIV,et/ou anti CD4, il est préférable de répéter plusieurs fois l'étape d'élimination par extraction, afin d'obtenir un titre pratiquement nul en anticorps anti-enveloppe HIV et/ou anti CD4.

En cas de présence de particules virales HIV dans le matériau, le procédé comporte également une étape d'élimination ou d'inactivation du virus.

Les donneurs ou les mères séropositifs peuvent être soit des personnes infectées par le virus HIV, soit des personnes vaccinées avec du virus HIV total inactivé ou provenant d'une souche non-infectieuse.

A titre d'exemple, on choisit du plasma, sang ou placenta de donneurs ou mères séropositifs pour HIV1, mais on peut également mettre en oeuvre le procédé pour HIV2 ou utiliser un mélange de matériaux relatifs à HIV1 et HIV2.

L'étape d'obtention des IgG et/ou IgM totales peut utiliser différents procédés bien connus, par exemple par fractionnement alcoolique de COHN pour le plasma ou de TAYLOR pour le placenta, ou encore par chromatographie. Les détails de ces fractionnements sont largement disponibles dans la littérature spécialisée.

L'étape de traitement spécifique d'inactivation virale anti-HIV, dans le cas où le matériau provient d'un donneur séropositif infecté par le virus sauvage, peut être, par exemple, un traitement à la béta-propiolactone ou par la chaleur ou par un solvant organique en présence éventuellement d'un détergent. De telles étapes d'inactivation sont maintenant usuelles ou décrites, par exemple dans les procédés de préparation d'immunoglobulines d'origine plasmatique ou placentaire.

(Voir : Inactivation of the human immunodeficiency viruses (HIV-1 and HIV-2) during the manufacturing of placental albumin and gammaglobulins. M. Grandgeorge and F. Pelloguin Transfusion. 1989 : 29-629.634).

L'étape d'élimination des anticorps anti-enveloppe de HIV ou anti CD4 peut être avantageusement effectuée par immunoadsorption en bain ou en colonne par mise en contact de la préparation d'immunoglobulines avec un support insoluble sur lequel on a fixé auparavant par toute méthode connue les protéines d'enveloppe gp120 (et/ou éventuellement gp41) et/ou leur précurseur gp160 (pour ce qui concerne HIV1) et/ou la protéine CD4. Les protéines peuvent, par exemple, être fixées sur un support d'agarose activé au bromure de cyanogène. Ces protéines peuvent être obtenues soit à partir de sources naturelles (HIV et lymphocytes), soit par recombinaison génétique.

Selon un autre procédé, on utilise du plasma, du sang ou des placentas de donneurs ou mères séropositifs HIV à la suite d'une vaccination avec des protéines isolées du virus HIV ou obtenues par recombinaison génétique, à l'exclusion des protéines d'enveloppe du virus. Dans ce procédé, les étapes d'élimination des anticorps anti-enveloppe HIV et/ou anti-CD4 sont alors omises.

La préparation obtenue par l'un des deux procédés précités est conditionnée pour être administrable par voie intramusculaire ou intraveineuse, par des étapes classiques de stabilisation, purification supplémentaire éventuelle, filtration stérile, et éventuellement lyophilisation.

Le médicament selon l'invention peut recevoir les applications suivantes :
- prévention de l'infection HIV chez les personnes à risque par séroprophylaxie,
- traitement adjuvant ou complémentaire d'une vaccination anti-HIV,
- traitement de l'infection HIV et prévention de sa manifestation clinique, le SIDA,
- traitement du SIDA.

### Exemple 1.

### Préparation de gammaglobulines anti HIV concentrées en anticorps anti-core et dépourvues d'anticorps anti-enveloppe

La matière de départ est une préparation de gammaglobulines humaines placentaires, riche en anticorps HIV, obtenue à partir d'un pool de placenta de femmes séropositives HIV, purifiée par une technique à l'alcool complétée par une étape d'inactivation virale par chauffage. Cette gammaglobuline est ajustée à une concentration de 50 g/litre en protéine, dans un tampon phosphate NaCl pH 7,4. La solution est titrée en ELISA (test Envacor Abbott HIV). Elle contient un titre de :
1/700 en anticorps anti-core (anti-noyau)
1/30 en anticorps anti-enveloppe.

Le titre est donné par la dernière dilution positive dans le test ELISA.
- 80 ml de cette solution sont filtrés sur une colonne contenant 3 mg d'antigène p25 fixé sur 1 ml de gel d'agarose.
- le filtrat obtenu contient l'essentiel des gammaglobulines ; ajusté à 50 g/l en protéine, il titre :
   1/30 en anticorps anti-core
   1/30 en anticorps anti-enveloppe.

La colonne est ensuite éluée à l'aide d'un tampon acide au glycocolle.

Cet éluat contient 13 mg de protéine,
Son titre ramené à une concentration en protéine de 50 g/l est:
de 1/120.000 en anticorps anti-core
négatif en anticorps anti-enveloppe.

Cet exemple illustre la possibilité de préparer une globuline anti-HIV riche en anticorps anti-core et dépourvue d'anticorps anti-enveloppe.

### Exemple 2.

### Préparation d'une gammaglobuline anti HIV déplétée en anticorps anti-enveloppe

La matière de départ est identique à celle de l'exemple 1. La globuline est ajustée à une concentration de 50 g/litre en protéine dans un tampon phosphate NaCl pH 7,4.

La solution est titrée en ELISA (test Envacor Abott HIV).

Elle a un titre de 1/700 en anticorps anti-core
de 1/30 en anticorps anti-enveloppe.
- 60 ml de cette solution sont filtrés sur une colonne contenant 4 mg d'antigène gp160 fixé sur 1 ml de gel d'agarose.
- Le filtrat est recueilli ; il contient l'essentiel des protéines. Son titre ajusté à 50 g/l en protéine est de
   1/500 en anticorps anti-core
   1/8 en anticorps anti-enveloppe.

Un deuxième passage de filtrat sur le support de colonne préalablement régénéré permet d'obtenir un second filtrat qui, ajusté à 50 g/l, titre :
1/500 en anticorps anti-core
1/3 en anticorps anti-enveloppe.

Après réalisation d'une 3ème filtration, sur le même support, dans les mêmes conditions que les deux premières, le 3ème filtrat obtenu ajusté à une concentration en protéine de 50 g/l contient toujours l'essentiel des protéines et possède un titre :
de 1/500 en anticorps anti-core
négatif en anticorps anti-enveloppe.
Cette préparation illustre la possibilité de préparer une gammaglobuline anti-HIV contenant notamment des anticorps anti-core mais dépourvue d'anticorps anti-enveloppe.

## Revendications (Revendications pour l'(les) Etat(s) contractant(s) suivant(s): AT, BE, CH, DE, FR, GB, IT, LI, LU, NL, SE)

1. Médicament constitué par ou comprenant une préparation de gammaglobulines IgG et/ou IgM d'origine humaine hyperimmunes anti-HIV dépourvue d'anticorps dirigés contre les glycoprotéines d'enveloppe du virus HIV et/ou d'anticorps anti CD4.

2. Procédé de préparation d'un médicament selon la revendication 1, caractérisé en ce que, à partir du plasma, sang ou placenta de donneurs ou mères séropositifs pour le virus HIV, on isole ou fractionne une préparation d'immunoglobulines totales IgG et/ou IgM, et on traite cette préparation pour en éliminer les anticorps anti-enveloppe HIV et/ou anti-CD4.

3. Procédé selon la revendication 2, caractérisé en ce que l'on met également en oeuvre une étape d'élimination ou d'inactivation du virus HIV.

4. Procédé selon l'une des revendications 2 et 3, caractérisé en ce que l'on prépare les immunoglobulines totales par un procédé choisi parmi le fractionnement alcoolique de COHN pour le plasma, le fractionnement de TAYLOR pour le placenta, et la chromatographie.

5. Procédé selon l'une des revendications 2 à 4, caractérisé en ce que l'on procède à l'élimination des anticorps anti-enveloppe de HIV et/ou anti CD4 par immunoadsorption en bain ou colonne.

6. Procédé selon la revendication 1, caractérisé en ce que l'on prépare les gammaglobulines totales IgG et/ou IgM à partir de plasma, sang ou placenta de donneurs ou mères séropositifs HIV à la suite d'une vaccination avec des protéines isolées du virus HIV ou obtenues par recombinaison génétique, à l'exclusion des protéines d'enveloppe du virus.

7. Procédé selon la revendication 5, caractérisé en ce que l'on effectue l'immuno-adsorption sur un support insoluble sur lequel on a fixé les protéines d'enveloppe, notamment gp120 et/ou gp160 et/ou la protéine CD4.

8. Procédé selon l'une quelconque des revendications 2 à 6, caractérisé en ce que l'on effectue une immuno-adsorption sélective sur un support insoluble sur lequel on a fixé un ou des antigènes HIV à l'exclusion des antigènes anti-enveloppe HIV et/ou anti-CD4.

9. Procédé selon la revendication 8, caractérisé en ce que l'antigène utilisé est p24,25.

## Revendications (Revendications pour l'(les) Etat(s) contractant(s) suivant(s): ES, GR)

1. Procédé de préparation d'un médicament constitué par ou comprenant une préparation de gammaglobulines IgG et/ou IgM d'origine humaine hyperimmunes anti-HIV dépourvue d'anticorps dirigés contre les glycoprotéines d'enveloppe du virus HIV et/ou d'anticorps anti CD4.caractérisé en ce que, à partir du plasma, sang ou placenta de donneurs ou mères séropositifs pour le virus HIV, on isole ou fractionne une préparation d'immunoglobulines totales IgG et/ou IgM, et on traite cette préparation pour en éliminer les anticorps anti-enveloppe HIV et/ou anti-CD4.

2. Procédé selon la revendication 1, caractérisé en ce que l'on met également en oeuvre une étape d'élimination ou d'inactivation du virus HIV.

3. Procédé selon l'une des revendications 1 et 2, caractérisé en ce que l'on prépare les immunoglobulines totales par un procédé choisi parmi le fractionnement alcoolique de COHN pour le plasma, le fractionnement de TAYLOR pour le placenta, et la chromatographie.

4. Procédé selon l'une des revendications 2 et 3, caractérisé en ce que l'on procède à l'élimination des anticorps anti-enveloppe de HIV et/ou anti CD4 par immunoadsorption en bain ou colonne.

5. Procédé selon la revendication 1, caractérisé en ce que l'on prépare les gammaglobulines totales IgG et/ou IgM à partir de plasma, sang ou placenta de donneurs ou mères séropositifs HIV à la suite d'une vaccination avec des protéines isolées du virus HIV ou obtenues par recombinaison génétique, à l'exclusion des protéines d'enveloppe du virus.

6. Procédé selon la revendication 4, caractérisé en ce que l'on effectue l'immuno-adsorption sur un support insoluble sur lequel on a fixé les protéines d'enveloppe, notamment gp120 et/ou gp160 et/ou la protéine CD4.

7. Procédé selon l'une quelconque des revendications 2 à 5, caractérisé en ce que l'on effectue une immuno-adsorption sélective sur un support insoluble sur lequel on a fixé un ou des antigènes HIV à l'exclusion des antigènes anti-enveloppe HIV et/ou anti-CD4.

8. Procédé selon la revendication 7, caractérisé en ce que l'antigène utilisé est p24,25.

## Claims (Claims for the following Contracting State(s): AT, BE, CH, DE, FR, GB, IT, LI, LU, NL, SE)

1. Medication constituted by or comprising a preparation of hyperimmune anti-HIV IgG and/or IgM gammaglobulins of human origin devoid of antibodies to glycoproteins of the HIV virus envelope and/or of anti-CD4 antibodies.

2. Process for the preparation of a medication according to claim 1, characterised in that one proceed to the isolation or fractionation of a preparation of total IgG and/or IgM immunoglobulins from plasma, blood or placenta of HIV-virus seropositive donors or mothers, said preparation being treated to eliminate the HIV anti-envelope and/or the anti-CD4 antibodies.

3. Process according to claim 2, characterised in that a further step is also carried out whereby said HIV-virus is removed or inactivated.

4. Process according to one of the claims 2 and 3, characterised in that the total immunoglobulins are prepared by a process selected from COHN's alcohol fractionation for plasma, TAYLOR's fractionation for placenta and chromatography.

5. Process according to one of the claims 2 to 4, characterised in that said HIV anti-envelope and/or anti-CD4 antibodies are eliminated by bath or column immunoadsorption.

6. Process according to claim 1, characterised by the preparation of total IgG and/or IgM gammaglobulins from plasma, blood, or placenta of HIV seropositive donors or mothers following a vaccination with proteins isolated from the HIV-virus or obtained through genetic recombination, excluding the virus envelope proteins.

7. Process according to claim 5, characterised in that the immunoadsorption is carried out on an insoluble support on which envelope proteins, particularly gp120 and/or gp160 and/or the CD4 protein have been immobilized.

8. Process according to any one of claims 2 to 6, characterised in that a selective immunoadsorption is carried out on an insoluble support on which a HIV antigen or HIV antigens, excluding the HIV anti-envelope and/or the anti-CD4 antigens, has been immobilized on a solid support.

9. Process according to claim 8, characterised in that the antigen used is p24,25.

## Claims (Claims for the following Contracting State(s): ES, GR)

1. Process for the preparation of a medication consisting of or comprising a preparation of hyperimmune anti-HIV IgG and/or IgM gammaglobulins of human origin devoid of antibodies to glycoproteins of the HIV virus envelope and/or anti-CD4 antibodies, characterised in that one proceed to the isolation or fractionation of a preparation of total IgG and/or IgM immunoglobulins from plasma, blood or placenta of HIV-virus seropositive donors or mothers, said preparation being treated to eliminate the HIV anti-envelope and/or the anti-CD4 antibodies.

2. Process according to claim 1, characterised in that a further step is also carried out whereby said HIV-virus is removed or inactivated.

3. Process according to one of the claims 1 and 2, characterised in that the total immunoglobulins are prepared by a process selected from COHN's alcohol fractionation for plasma, TAYLOR's fractionation for placenta and chromatography.

4. Process according to one of the claims 2 and 3, characterised in that said HIV anti-envelope and/or anti-CD4 antibodies are eliminated by bath or column immunoadsorption.

5. Process according to claim 1, characterised by the preparation of total IgG and/or IgM gammaglobulins from plasma, blood, or placenta of HIV seropositive donors or mothers following a vaccination with proteins isolated from the HIV-virus or obtained through genetic recombination, excluding the virus envelope proteins.

6. Process according to claim 4, characterised in that the immunoadsorption is carried out on an insoluble support on which envelope proteins, particularly gp120 and/or gp160 and/or the CD4 protein have been immobilized.

7. Process according to any one of claims 2 to 5, characterised in that a selective immunoadsorption is carried out on an insoluble support on which a HIV antigen or HIV antigens, excluding the HIV anti-envelope and/or the anti-CD4 antigens, has been immobilized on a solid support.

8. Process according to claim 7, characterised in that the antigen used is p24,25.

## Patentansprüche (Patentansprüche für folgende(n) Vertragsstaat(en): AT, BE, CH, DE, FR, GB, IT, LI, LU, NL, SE)

1. Medikament, bestehend aus einem oder umfassend ein Präparat von Anti-HIV-Hyperimmun-IgG- und/oder -IgM-Gammaglobulinen menschlichen Ursprungs, frei von Antikörpern, die gegen die Hüllglycoproteine des HIV-Virus gerichtet sind, und/oder von Anti-CD4-Antikörpern.

2. Verfahren zur Herstellung eines Medikaments nach Anspruch 1, dadurch gekennzeichnet, daß man ausgehend vom Plasma, Blut oder von der Plazenta von bezüglich des HIV-Virus seropositiven Spendern oder Müttern ein Präparat von Gesamt-IgG- und/oder -IgM-Immunglobulinen isoliert oder fraktioniert und dieses Präparat behandelt, um die Anti-HIV-Hüllen- und/oder Anti-CD4-Antikörper daraus zu entfernen.

3. Verfahren nach Anspruch 2, dadurch gekennzeichnet, daß man ebenfalls einen Schritt zur Entfernung oder Inaktivierung des HIV-Virus durchführt.

4. Verfahren nach einem der Ansprüche 2 und 3, dadurch gekennzeichnet, daß man die Gesamtimmunglobuline durch ein Verfahren herstellt, das aus der alkoholischen Plasma-Fraktionierung nach COHN, der Plazenta-Fraktionierung nach TAYLOR und der Chromatographie ausgewählt ist.

5. Verfahren nach einem der Ansprüche 2 bis 4, dadurch gekennzeichnet, daß man die Entfernung der Anti-HIV-Hüllen- und/oder Anti-CD4-Antikörper durch Immunadsorption im Bad oder an der Säule durchführt.

6. Verfahren nach Ansprüch 2, dadurch gekennzeichnet, daß man die Gesamt-IgG- und oder -IgM-Gammaglobuline anschließend an eine Impfung mit Proteinen, die aus dem HIV-Virus isoliert oder durch genetische Rekombination erhalten worden sind, ausschließlich der Hüllproteine des Virus, ausgehend von Plasma, Blut oder Plazenta von HIV-seropositiven Spendern oder Müttern herstellt.

7. Verfahren nach Anspruch 5, dadurch gekennzeichnet, daß man die Immunadsorption auf einem unlöslichen Träger bewirkt, auf dem man die Hüllproteine, insbesondere gp120 und/oder gp160, und/oder das Protein CD4 fixiert hat.

8. Verfahren nach irgendeinem der Ansprüche 2 bis 6, dadurch gekennzeichnet, daß man eine selektive Immunadsorption auf einem unlöslichen Träger bewirkt, auf dem man HIV-Antigen(e), ausschließlich der Anti-HIV-Hüllen- und/oder Anti- CD4-Antigene, fixiert hat.

9. Verfahren nach Anspruch 8, dadurch gekennzeichnet, daß es sich bei dem verwendeten Antigen um p24,25 handelt.

## Patentansprüche (Patentansprüche für folgende(n) Vertragsstaat(en): ES, GR)

1. Verfahren zur Herstellung eines Medikaments, bestehend aus einem oder umfassend ein Präparat von Anti-HIV-Hyperimmun-IgG- und/oder -IgM-Gammaglobulinen menschlichen Ursprungs, frei von Antikörpern, die gegen die Hüllglycoproteine des HIV-Virus gerichtet sind, und/oder von Anti-CD4-Antikörpern, dadurch gekennzeichnet, daß man ausgehend vom Plasma, Blut oder der Plazenta von bezüglich des HIV-Virus seropositiven Spendern oder Müttern ein Präparat von Gesamt-IgG- und oder -IgM-Immunglobulinen isoliert oder fraktioniert und dieses Präparat behandelt, um die Anti-HIV-Hüllen- und/oder Anti-CD4-Antikörper zu entfernen.

2. Verfahren nach Anspruch 1, dadurch gekennzeichnet, daß man ebenfalls einen Schritt zur Entfernung oder Inaktivierung des HIV-Virus durchführt.

3. Verfahren nach einem der Ansprüche 1 und 2, dadurch gekennzeichnet, daß man die Gesamtimmunglobuline durch ein Verfahren herstellt, das aus der alkoholischen Plasma-Fraktionierung nach COHN, der Plazenta-Fraktionierung nach TAYLOR und der Chromatographie ausgewählt ist.

4. Verfahren nach einem der Ansprüche 2 und 3, dadurch gekennzeichnet, daß man die Entfernung der Anti-HIV-Hüllen- und/oder Anti-CD4-Antikörper durch Immunadsorption im Bad oder an der Säule durchführt.

5. Verfahren nach Anspruch 1, dadurch gekennzeichnet, daß man die Gesamt-IgG- und/oder -IgM-Gammaglobuline anschließend an eine Impfung mit Proteinen, die aus dem HIV-Virus isoliert oder durch genetische Rekombination erhalten worden sind, ausschließlich der Hüllproteine des Virus, ausgehend von Plasma, Blut oder Plazenta von HIV-seropositiven Spendern oder Müttern herstellt.

6. Verfahren nach Anspruch 4, dadurch gekennzeichnet, daß man die Immunadsorption auf einem unlöslichen Träger bewirkt, auf dem man die Hüllproteine, insbesondere gp120 und/oder gp160, und/oder das Protein CD4 fixiert hat.

7. Verfahren nach irgendeinem der Ansprüche 2 bis 5, dadurch gekennzeichnet, daß man eine selektive Immunadsorption auf einem unlöslichen Träger bewirkt, auf dem man HIV-Antigen (e), ausschließlich der Anti-HIV-Hüllen- und/oder Anti-CD4-Antigene fixiert hat.

8. Verfahren nach Anspruch 7, dadurch gekennzeichnet, daß es sich bei dem verwendeten Antigen um p24, 25 handelt.
